# EUROPEAN PATENT SPECIFICATION

(11) **EP 1 029 246 B1**
(45) Date of publication and mention of the grant of the patent: **04.08.2004**
(21) Application number: 98956035.4
(22) Date of filing: 12.11.1998
(51) Int. Cl.: G01N 33/68, G01N 33/53, G01N 33/569, C07K 1/06, A61K 31/315, B01L 3/00, A61K 39/395, A61K 39/40, A61K 39/42

(54) **A METHOD AND DEVICE FOR PREVENTING IMMUNOGLOBULIN DEGRADATION IN EXTERNAL SECRETIONS**
EIN VERFAHREN UND GERÄT ZUR VORBEUGUNG VON IMMUNGLOBULINDEGRADATION IN EXTERNEN SEKRETIONEN
PROCEDE ET DISPOSITIF PERMETTANT D'EMPECHER LA DEGRADATION DE L'IMMUNOGLOBULINE DANS DES SECRETIONS EXTERNES

(30) Priority: 14.11.1997 NO 975229
(43) Date of publication of application: 23.08.2000
(73) Proprietor: UNIFOB STIFTELSEN UNIVERSITETSFORSKNING BERGEN, 5020 Bergen (NO)
(72) Inventor: GREWAL, Harleen, M., S., N-5030 Land s (NO)
(74) Representative: Plougmann & Vingtoft A/S
(86) International application number: PCT/NO1998/000338
(87) International publication number: WO 1999/026068

(56) References cited:
- US-A- 5 094 845
- PATENT ABSTRACTS OF JAPAN vol. 18, no. 555 (P-1816), 21 October 1994 & JP 06 201684 A (SUTEMU KK)
- MEDLINE, Washington DC USA; abstract no. 98416007, see abstract XP002097879 & H. VETVIK ET AL.: "Mucosal antibodies can be measured in air-dried samples of saliva and feces." JOURNAL OF IMMUNOLOGICAL METHODS, vol. 215, no. 1, 1 June 1998, pages 163-172, Amsterdam NL

## Description

### FIELD OF THE INVENTION

The present invention relates to the employment of a method for inhibiting the degradation of substances in external secretions. The inventive compounds protect the proteins from the otherwise extensive degradation that takes place in such secretions, including in feces. Specifically, the invention relates to a method which substantially protects immunoglobulins in fecal samples. Collection devices are also provided.

### BACKGROUND OF THE INVENTION

Infectious diseases, in particular enteric infections such as diarrhoea and typhoid fever constitute a serious health problem throughout the world. Protection against these infections is provided by the gastrointestinal immune system, and considerable efforts have been made to develop oral vaccines that may stimulate the mucosal immune system in the gut. Bacterial infections of the gastrointestinal tract, as well as oral vaccines, produce a secretory IgA immune response in the intestine, which may be accompanied by a more widespread response with specific secretory immunoglobulin A (SIgA) detectable in the external secretions of other distant mucosal surfaces. This pattern of response has been reported to occur without the corresponding presence of detectable specific antibodies in serum. The development of effective vaccines against infectious diseases requires the existence of reliable and meaningful immunological correlates of protection against disease.

For immune mediated protection against enteric diseases, the induction of strong local intestinal immune responses is required. An assessment of the immunogenicity of orally administered enteric vaccines has frequently relied on indirect measurements of intestinal antibodies, such as the determination of levels of specific antibody in serum, colostrum or saliva and more recently, by the quantitation of specific antibody producing circulating peripheral blood lymphocytes, i.e. antibody secreting cells (ASCs). In one study (Forrest, B. D., Infection and Immunity, 1992, Vol. 60: 2023-2029) it has been shown in human volunteers vaccinated with orally administered typhoid vaccines, that, despite substantial specific intestinal-IgA responses, the specific salivary-IgA responses in the samples collected concurrently with intestinal fluid were of very low magnitude. Thus, the available methods for the evaluation of antigen-specific immune responses in the intestine include; the measurement of IgA in jejunal fluid by intestinal intubation or in intestinal lavage fluids, and by the quantitation of ASCs in peripheral blood. These methods are not applicable to large-scale immunogenicity studies. Moreover, intestinal intubation and lavage methods are very labour-intensive, invasive (intubation) and time-consuming procedures that are highly unpleasant for the individuals undergoing them and are contradicted in an important population group, namely children. Furthermore, clinical studies and experiments in animals show that measurements of various components of the systemic immune system (serum antibodies, cytokines, circulating cells) are rarely reliable indices of local mucosal immunity. Therefore, simple and reliable methods for the measurement of local mucosal immune responses are crucial for the evaluation of the natural history of enteric infectious diseases and for the development of enteric vaccines.

The use of external secretions for the analysis of mucosal antibodies is impeded by the fact that substances present in these secretions cause antibody degradation. This is especially a problem for fecal samples which contain various substances that degrade or inactivate immunoglobulins, including enzymes (proteases) produced for instance by bacteria in the intestinal tract that degrade fecal antibodies (coproantibodies). Since 80% of the dry weight of feces from a healthy human consists of bacteria, the degradation of antibodies is substantial if the coproantibodies are not suitably protected. The exploitation of fecal IgA for the monitoring of mucosal immune responses is highly relevant for the evaluation of orally administered enteric vaccines. In contrast to blood and urine for which standard devices are available for the collection, transport, protection, and detection of immunoglobulins, there are no devices for unaltered fecal samples.

Various investigations aimed at analyzing immunoglobulins extracted from fluids of the oral cavity have been made, resulting in the following patents:

US 5,103,836 discloses a method and device for collecting immunoglobulins from the oral cavity by inserting an absorbent pad impregnated with the salts of a hypertonic solution to enhance the recovery of said substances. The pad may be immersed in a preservative solution, e.g. containing chlorhexidine gluconate.

US 5,022,409 discloses a method and kit for the collection of immunoglobulins from the oral cavity by rinsing the oral cavity with a hypertonic solution.

### BRIEF SUMMARY OF THE INVENTION

In order to eliminate or greatly reduce the problems inherent in using fecal samples for antibody detection and quantitation, the present invention provides a method for the collection, storage and extraction of immunoglobulins from fecal samples, the extracted immunoglobulins being then suitable for use in immunoassays. The method involves the collection of fecal samples in a solution which prevents the degradation of immunoglobulins to an extent that allows the extraction and subsequent use of the extracted immunoglobulins in basic antigen-antibody testing techniques for the screening of diseases as well as provides a valuable tool for evaluating the immunogenicity of potential orally administered vaccines.

The above objectives of the present invention are obtained by collecting fecal samples in a solution containing an optimum concentration of zinc gluconate. Coproantibodies suitable for use in relevant immunoassays are then extracted from the fecal samples by a simple 2-step extraction procedure as detailed below.

### BRIEF DESCRIPTION OF THE FIGURES.

Figure 1 shows the fecal collection device, which includes the protective solution of the present invention.
Figure 2 shows an alternative fecal collection device.
Figure 3 shows an embodiment of an alternative collection device, i.e. a syringe with several independent compartments.
Figure 4 shows the effect of zinc gluconate on the inhibition of the degradation of anti-Borrelia IgM antibodies in feces (spiked samples).
Figure 5 shows the effect of various normotonic solutions on the inhibition of the degradation of anti-Borrelia IgM antibodies in feces (spiked samples).
Figure 6 shows the ability of zinc gluconate to inhibit the degradation of anti-CT IgA.
Figure 7 shows the mean and 95% confidence intervals of log transformed anti-LT IgA indices in children known and not known to be infected with LT.ETEC.

### DETAILED DESCRIPTION OF THE INVENTION

The present invention is concerned with a method of inhibiting the degradation of substances in external secretions wherein the method comprises the addition of a solution comprising zinc gluconate to external secretions.

One aspect of the present invention is concerned with the collection of fecal samples in a solution that significantly prevents the degradation of coproantibodies. The compound or the solution prepared from this compound is harmless and non-toxic.

The compound of the present invention significantly protect fecal antibodies from breakdown for several hours at 20-37°C. The fecal samples are collected in disposable single-use tubes or syringes containing this protective solution. This method thus allows safe transport of the fecal samples to the laboratory where the fecal antibodies can be extracted from the sample for diagnostic and research purposes.

Immunoglobulins in feces undergo substantial degradation within 1-2 hours at 20-37°C. Without the use of the protective substance in accordance with the present invention, cumbersome procedures such as the immediate refrigeration of samples upon collection and subsequent storage at -70°C within 4 hours of sample collection in hospitals or institutions are required in order to substantially prevent immunoglobulin degradation in feces. Furthermore. if feces is collected at home, the samples have to be immediately frozen in domestic freezers until they can be collected and transported to the hospital/laboratory in car freezers and then stored at -70°C (Coulson, B.S. et al Journal of Clinical Microbiology, 1990;28:1367-1374). These methods are cumbersome, labor-intensive and expensive and are not feasible for large scale vaccine studies nor for the routine diagnosis of infectious diseases, especially in developing countries.

The method and the device provided is particularly attractive since the method involves the collection of external secretions, the inherent risks with the collection of blood and serum are thus circumvented. Moreover, the fact that the collection procedure is non-invasive makes it more attractive for the sample donor. Most importantly, as outlined above, measuring mucosal antibodies is more relevant than estimating serum antibodies for the evaluation of a local mucosal immune response. This is especially true for several important infections affecting the mucosal systems, i.e. the respiratory, gastrointestinal and the genito-urinary tracts.

In extensive preliminary studies using the device in accordance with the present invention, we have shown that coproantibodies, such as IgA and IgM antibodies are significantly protected from the otherwise extensive degradation which takes place in feces stored for several hours at 20-37°C. Furthermore, the coproantibodies are, after having been extracted from the fecal samples by a simple 2-step procedure stable for several months (> 4 months) at -20°C. We assume that the mechanism of action of the compound is by the inhibition of proteolytic degradation of immunoglobulins. However, the exact mechanism of action needs to be established.

To simplify the collection of fecal samples using the immunoglobulin stabilizing compound of the present invention, several devices are provided. One embodiment of such a device can include a container, e.g. a plastic tube, containing the protective solution, a lid which can be screwed onto the container to seal its contents, and optionally a spoon attached to the lid in order to facilitate the collection of the fecal sample. Another embodiment of such a device comprises a syringe optionally divided into a plurality of separate compartments.

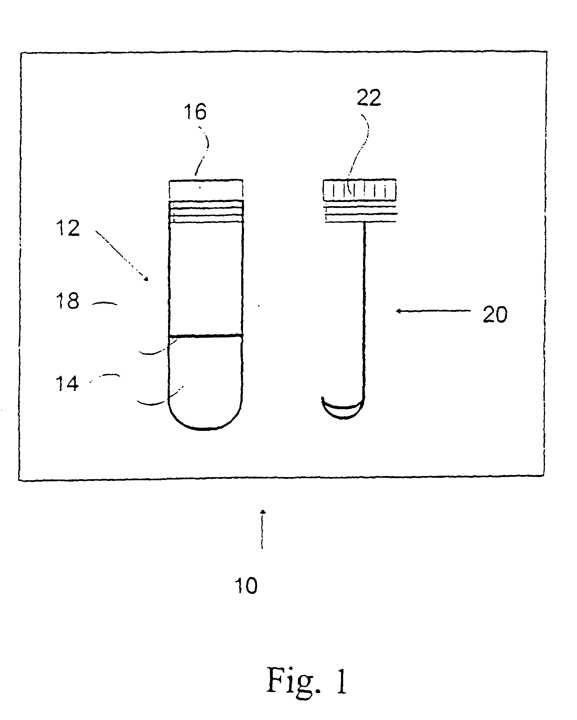

Reference is now made to figure 1 wherein a preferred embodiment of a device 10 in accordance with the present invention is shown. The device 10 consists of a container 12 and a spoon 20. The container 12 includes a solution 14 comprising the compound of the present invention, optionally in combination with additional preservatives. Alternatively, this solution can be added to the container after the fecal sample has been transferred to the container 12. The container 12 is closed by means of a lid or cap 16. Further, the device 10 can preferably be provided with a membrane 18 encasing the solution 14 and thus preventing the accidental spillage of solution 14, before the fecal sample is introduced into container 12. Optionally, the device 10 consists of a spoon 20 for the collection of the fecal sample. Preferably, the spoon 20 is provided with a cap or stopper 22 adapted to fit the container 12. The stopper 22 will thus securely seal the container 12. By inserting the spoon 20 in the container 12 the membrane 18 is pierced and the fecal sample is brought in contact with the solution 14. The container 12 is sealed and the sample is dispersed in the solution 14 by vigorously shaking the container 12.

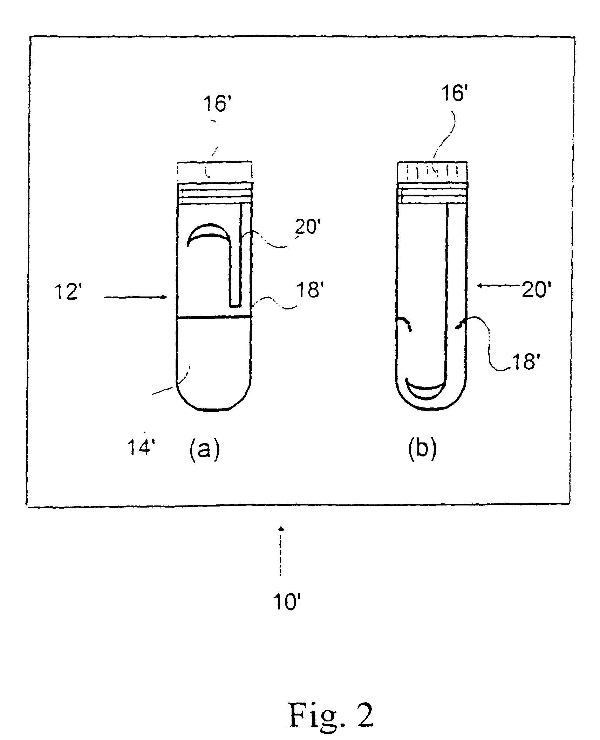

Alternatively, with reference to figure 2, the collection device 10' includes a container 12' containing the protective solution 14' of the present invention. The device 10' is provided with a membrane 18' encasing the solution 14', thus preventing the accidental spillage of solution 14', before the fecal sample is introduced into container 12'. The device 10' has a lid 16' to which is attached a foldable or collapsible spoon 20'. The fecal sample is collected by first opening the device 10' and then opening the spoon 20'. The insertion of the spoon 20' pierces the membrane 18' (Figure 2b), thus allowing the fecal sample to come in contact with the protective solution 14'.

The container, the cap and the spoon are made of any suitable material such as polyethylene, polypropylene etc. The membrane can be made of materials such as PVC or other plastic materials. The membrane is impermeable to an aqueous solution.

An embodiment of the device as shown in figure 1 or 2 comprises a container that is adapted to fit directly into a centrifuge.

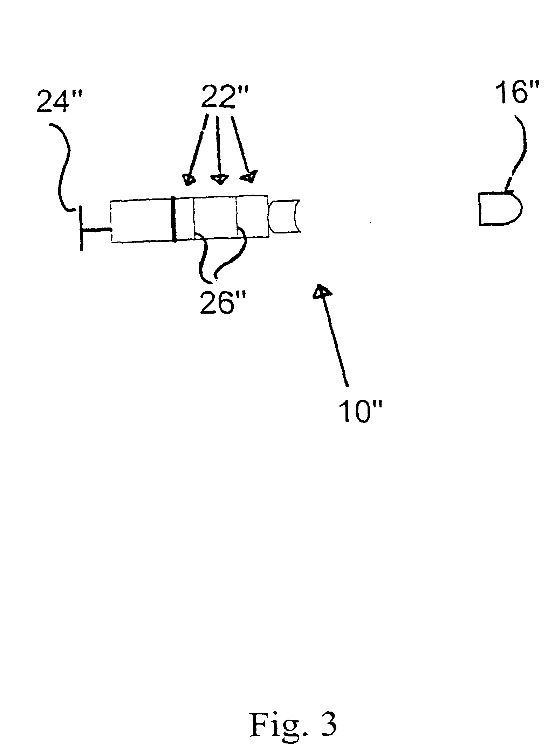

A further embodiment of a device in accordance with the invention is shown in figure 3. This is actually a syringe 10" provided with one or several compartments 22" separated by separating membranes 26". The syringe is further provided with a piston 24". A most preferred embodiment of this syringe contains zinc gluconate in dry form, i.e. as a powder, in a first compartment, and a suitable buffer solution such as for example PBS in a second compartment. Optionally, the syringe can be provided with a third empty compartment, and/or optionally a fourth compartment containing additional preservatives. The syringe is also provided with a removable cap, e.g. a screw cap which securely seals the syringe 10".

Upon storage the individually compounds are kept separated from each other. It is thus possible to use zinc gluconate in a dry powder form. While pulling the piston in order to suck the fecal sample into the syringe, the piston will break the separating membrane(s) and this thus allows mixing of the various components in the syringe, i.e. the fecal sample will by shaking the syringe mix with the dry zinc gluconate and the buffer solution. The use of dry zinc gluconate will with respect to zinc gluconate in solution increase the shelf-life of the inventive compound considerable thus making it possible to store the syringes for several years before use.

The volume of the container or syringe may vary. In a preferable embodiment 2 ml of a solution of zinc gluconate is added to the collection device for the collection of fecal samples or fecal swabs. Accidental spillage of the protective solution while using the collection device, is prevented by the presence of the thin membrane. The collection devices are preferably kept at +4°C until use.

The concentrations of the compound in the solution can be in the range of from 0.01 to 20 % w/v, preferably from 5 to 15 % w/v. A preferred embodiment of the device of the invention comprises a 10 % (w/v) solution of zinc gluconate.

Preferably additional preservative compounds can be added to the solution. Compounds contemplated as a preservative include anti-bacterial agents, anti-fungal agents, bacteriostatic agents, fungistatic agents and enzyme inhibitors. Benzoic acid and sorbic acid are examples of antifungicides. Bactenocides includes thiomersal, phenyl mercuric acetate, phenyl mercuric nitrate and sodium azide. Protease inhibitors include but are not restricted to Bestatin, AEBSF, Aprotinin and Leupeptin. Other preservatives commonly used are ethyl alcohol, chlorhexidine gluconate and detergents such as benzalkonium chloride. The preservatives can be used in the range of about 0.01 % w/v to about 0.5% w/v.

Approximately one level spoon of a freshly passed fecal sample or a recently collected fecal swab is placed into the collection device or syringe, the lid is closed tightly and the device is shaken vigorously to disperse the feces into the protective solution. The collected samples are then placed in a refrigerator until the samples can be sent to a laboratory for further processing, a process which in certain field conditions can take up to 6-7 hours. At the laboratory the coproantibodies are extracted after addition of standard commercially available protease inhibitors (Bestatin, AEBSF, Aprotinin, Leupeptin) followed by centrifuging at 13 500 rpm for 15 min. The supernatants contain the protected coproantibodies and can be analyzed directly in relevant immunoassays or stored at -20°C for later analysis. Because zinc gluconate mediate a substantial protection of the antibodies (see below), it is highly probable that the addition of commercial protease inhibitors during terminal processing of the fecal samples at the laboratory is unnecessary. Further studies will clarify this issue.

Thus, an embodiment of the invention relates to a method of analyzing immunoglobulins in fecal samples wherein the method comprising the steps of:
i) collecting the feces.
ii) mixing the fecal samples with a solution comprising zinc gluconate, and vigorously mixing the sample and the solution, wherein the compound in the solution is in an effective concentration to inhibit the degradation of immunoglobulins and thus enabling the recovery of high concentration of immunoglobulins, and
iii) analyzing the immunoglobulins by enzyme-linked immunoabsorbent assay (ELISA) or any other assay for the detection of immunoglobulins such as radioimmuno assays, Western Blot assay etc.

The term "inhibiting the degradation of substances" is intended to mean that the solution of the present invention is capable of preserving or stabilizing the structure of said substances sufficiently to be able to analyze the said substances in basic immunological detection techniques.

The term "fecal sample" indicates a swab that has briefly been inserted into the anal opening or a sample of freshly passed feces.

The term "coproantibodies" indicates antibodies present in feces such as IgA, IgM, IgG and IgE.

### EXAMPLES

The following examples are offered to illustrate, but not to limit the present invention in any way.

In example numbers 1-5, fecal samples are spiked with a single analyte of interest, i.e. a predetermined amount of a high titered anti-Borrelia IgM serum is added to fecal samples in order to determine the extent to which the protective solution of the present invention prevents the degradation of the particular analyte added.

### Example 1

### Zinc gluconate inhibits the degradation of IgM

50 µl of a high titer anti-Borrelia IgM serum obtained from a patient with acute Borreliosis was added to 3 tubes each containing 50 mg of a fecal sample from a healthy volunteer. Following vortexing for 30 seconds, the tubes were incubated at 30°C for 5 hours in the presence or absence of 150 µl of 10% zinc gluconate (Gluconal® ZN-G; Glucona B.V., Veendam, The Netherlands). Following incubation, 150 µl of a protease inhibitor cocktail (0.2 mM 4-{2-aminoethyl}benzenesulfonylfluoride [Calbiochem], 1 µg of aprotinin [Sigma] per ml, 10 µM leupeptin [Sigma], and 3.25 µM bestatin [Boehringer Mannheim, Indianapolis, USA]) was added. After vortexing, the tubes were centrifuged at 13,000 x *g* for 10 min. The supernatants were stored at -70°C or immediately tested in a commercially available anti-Borrelia IgM ELISA (IDELA™ Borrelia burgdorferi IgM, DAKO Diagnostics Ltd., Cambridgeshire, UK). For the purpose of comparison, 50 µl of anti-Borrelia IgM serum was run in the immunoassay.

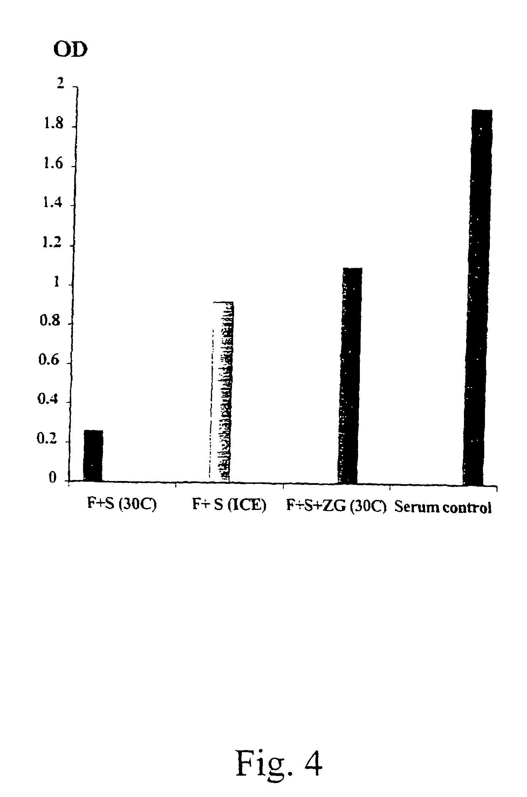

The results are given in figure 4, Y-axis: ELISA results (O.D. value); X-axis: F+S; feces plus anti-Borrelia IgM serum incubated at 30°C or on ice for 5 hours, F+S+ZG; feces plus anti-Borrelia IgM serum plus zinc gluconate incubated at 30°C for 5 hours. All values are depicted at a 1:1620 dilution of the original supernatant/control serum.

As indicated in the figure 4, the addition of a 10% solution of zinc gluconate during the incubation of the fecal samples at 30°C for 5 hours, substantially inhibits the degradation of the added analyte i.e. the anti-Borrelia IgM antibodies. It is expected that the degradation of proteins such as antibodies is related to the incubation temperature, i.e. the degradation is higher at 30°C than at 0°C. It is thus surprising that antibody degradation at 30°C with the addition of zinc gluconate is lower than at 0°C without the addition of zinc gluconate. The mechanism of action is not fully established but it is anticipated that zinc gluconate inhibits various proteinases, i.e. protein hydrolyzing enzymes. Thus, it is anticipated that zinc gluconate will also inhibit the degradation of other immunoglobulins, and possibly also other proteins.

### Example 2

### Long term stability of IgM antibodies extracted from spiked fecal samples

The fecal extracts described in Example 1 were stored at -70°C for 4 months, and then re-analyzed in the anti-Borrelia IgM ELISA to check the long term stability of the antibodies stored in the presence or absence of 10% zinc gluconate.

The results are given in table I.

**Table I**

| **Sample, incubation temperature and sample dilution** | **Initial ELISA, O.D.** | **ELISA O.D. after 4 months** |
|---|---|---|
| F+S (30°C): | | |
| 1/60 | 1.90 | 1.65 |
| 1/180 | 1.73 | 1.43 |
| 1/540 | 1.00 | 0.76 |

| F+S (ice): | | |
|---|---|---|
| 1/60 | 2.38 | 2.18 |
| 1/180 | 2.42 | 2.22 |
| 1/540 | 1.82 | 1.89 |

| F+S+ZG (30°C): | | |
|---|---|---|
| 1/60 | 2.59 | 2.48 |
| 1/180 | 2.57 | 2.40 |
| 1/540 | 2.08 | 1.75 |

| Control serum | | |
|---|---|---|
| 1/60 | 2.82 | 2.75 |
| 1/180 | 2.93 | 2.67 |
| 1/540 | 2.48 | 2.58 |
| F-S: feces plus anti-Borrelia IgM serum incubated at 30°C or on ice for 5 hours. F-S+ZG: feces plus anti-Borrelia IgM serum plus zinc gluconate incubated at 30°C for 5 hours. | | |

This study demonstrates that the IgM antibodies extracted from fecal samples are relatively stable following long term storage at -70°C.

### Example 3

### The inhibiting effect of normotonic zinc gluconate (319 mOsm/L) on the degradation of IgM antibodies.

25 µl of a high titred anti-Borrelia IgM serum obtained from a patient with acute Borreliosis was added to 5 tubes, each containing 50 mg of a fecal sample from a healthy volunteer. Following vortexing for 30 seconds, 500 µl of the normotonic (319 mOsm/L) solutions (i) 1 X PBS (7,7 mM Na₂HPO₄, 2,99 mM NaH₂PO₄, 145 mM NaCl), (ii) 64.5 mM zinc gluconate, (iii) 64,5 mM MgCl₂, (iv) 64.5 mM magnesium gluconate or (v) 96.9 mM NaCl, the latter four all dissolved in 1/3 X PBS to obtain the same osmolarity as 1 X PBS, i.e. 319 mOsm/L was added to each of the five tubes. Following vortexing for 30 seconds, the tubes were subsequently incubated at 30°C for 5 hours. Thereafter, 250 µl of a protease inhibitor cocktail (see above) was added to each of the 5 tubes. Solid matter was suspended by vortexing for another 30 seconds followed by centrifugation at 13,000 x *g* for 10 min. The supernatants were stored at -70°C prior to examination or immediately tested in a commercially available anti-Borrelia IgM ELISA. For the purpose of comparison, 25 µl of the anti-Borrelia IgM serum was run in the immunoassay.

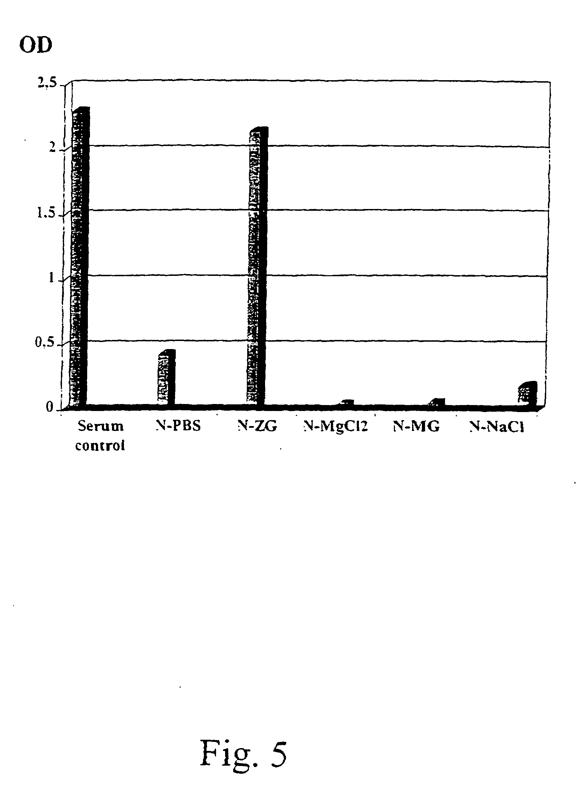

The results are given in Figure 5, Y-axis: ELISA results (O.D. value), X-axis: N-PBS; feces plus normotonic PBS, N-ZG; feces plus anti-Borrelia IgM serum plus normotonic zinc gluconate-PBS, N-MgCl2; feces plus anti-Borrelia IgM serum plus normotonic MgCl₂-PBS, N-MG; feces plus anti-Borrelia IgM serum plus normotonic magnesium gluconate-PBS, N-NaCl; feces plus anti-Borrelia IgM serum plus normotonic NaCl-PBS.

This study shows that 64.5 mM zinc gluconate mixed to normotonicity (319 mOsm/l) in 1/3 X PBS substantially inhibits the degradation of anti-Borrelia IgM at 30°C in feces. Moreover, it shows that this protective activity is greater than that conferred by the tonicity of the salts alone and, lastly, that, of the examined compounds, zinc gluconate is the most efficient in conferring such protection.

### Example 4

50 milligrams of feces and 25 µl of anti-Borrelia IgM serum were incubated at 33°C for 3 hours with each of the substances described in table 2. Following incubation, the fecal samples were processed and the extracts were obtained as described in example 3.

**Table 2**

| **Substance*** | **Dilution in ELISA** | **ELISA OD** |
|---|---|---|
| Zinc gluconate | 1/3000 | 0.360 |
| Manganese chlonde | 1/3000 | 0.184 |
| Sodium chloride | 1/3000 | 0.043 |
| Sodium gluconate | 1/3000 | 0.087 |
| Copper sulphate | 1/3000 | 0.018 |
| Copper acetate | 1/3000 | 0.042 |
| Chlorhexidine gluconate | 1/3000 | 0.003 |
| Phosphate buffered saline | 1/3000 | 0.014 |

| | | |
|---|---|---|
| * The concentrations of all cations were 0.219M | | |

The experiment shows that of the substances tested, zinc gluconate gives the best protection.

### Example 5

50 milligrams of feces and 25 µl of anti-Borrelia IgM serum was incubated at 37°C for 4 hours with equimolar concentrations of zinc gluconate, zinc chloride and zinc acetate (zinc concentration = 0.219M).

**Table 3**

| **Substance** | **Dilution in ELISA** | **ELISA OD** |
|---|---|---|
| Zinc gluconate | 1/1450 | 0.910 |
| Zinc chloride | 1/1450 | 0.011 |
| Zinc acetate | 1/1450 | 0.281 |

Thus, the experiment shows that the specific zinc compound, i.e. zinc gluconate, significantly protects the added analyte, i.e. anti-Borrelia IgM antibodies, from degradation during prolonged incubation at high ambient temperatures.

### Example 6

### Ability of zinc gluconate to inhibit the degradation of fecal IgA

A Swedish volunteer was recruited after giving informed consent. The protocol was approved by the Swedish National Pharmaceutical Agency and the Human Research Ethical Committee of the medical faculty, University of Gøteborg, Sweden. The volunteer was given three doses at 2-week intervals of the prototype oral enterotoxigenic *Escherichia coli* vaccine (Åhren *et al*., 1993, Vaccine 11, 929) containing a mixture of cholera B subunit (CTB) and formalin-killed *E. coli* bacteria. 23 days following the initial immunization, a fecal sample from the volunteer was collected in a small plastic cup. 50 mg of this sample was transferred to three 1.5 ml micro-centrifuge tubes each containing 300 µl of PBS or zinc gluconate. The tubes were then incubated at 33°C for 5 hours. Extraction was performed as mentioned above. Quantitation of specific anti-CTB IgA in the fecal extracts was determined by a GM1-ELISA method as previously described (Åhren *et al*., 1993, Vaccine 11, 929).

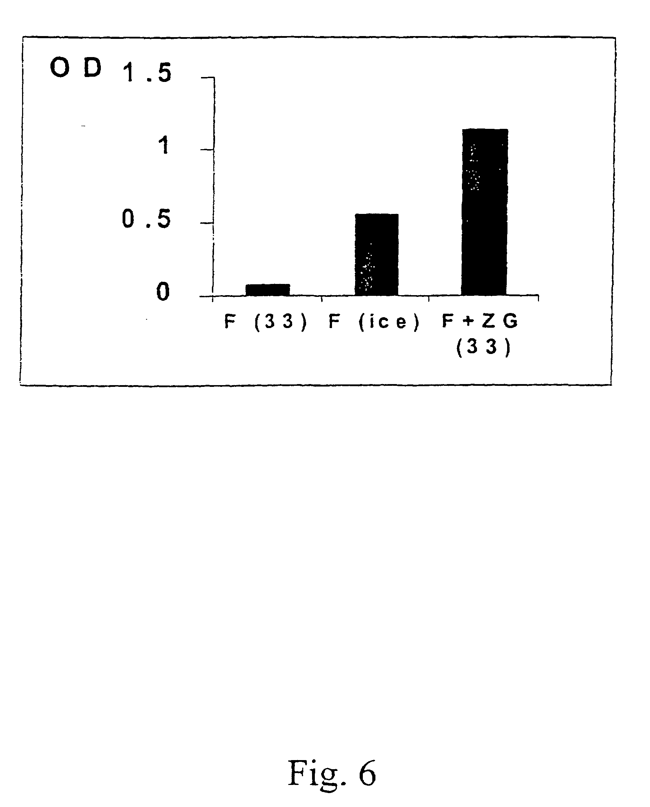

The results are depicted graphically in figure 6: Y-axis: ELISA results (O.D. value); X-axis: F; feces from a volunteer collected 23 days after receiving two doses of the prototype oral cholera vaccine, incubated at 33°C for 5 hours or on ice for 5 hours. F+ZG; feces from a volunteer collected in a zinc gluconate solution and incubated at 33°C for 5 hours. The feces was collected 23 days after receiving two doses of the prototype oral cholera vaccine

The results show that zinc gluconate can also significantly inhibit the degradation of IgA. Moreover, the biological activity of the IgA molecules, namely their capacity to bind antigen (CTB) is preserved. Thus, it is reasonable to believe that other immunoglobulins will also be protected from similar degradation by the compound of the present invention.

### Example 7

### Field testing of the collection device in Guinea-Bissau in November-December 1996.

Fecal specimens from 19 children (0-5 years) infected with enterotoxigenic *E. coli* producing the LT toxin (LT-ETEC) during the last 40 days and 15 children (0-5 years) not known to be infected with LT-ETEC during the same period were examined. The fecal samples were collected by field workers in tubes containing 10% zinc gluconate in PBS. Since the field workers collected the samples from the individual houses, it took on an average 5-6 hours at high ambient temperatures (28-33°C) before the samples were processed in the laboratory in Bissau. Processing and extraction was performed as described above. Quantitation of specific anti-LT IgA and total IgA in the fecal extracts was performed by a CTB-GM1-ELISA and a modified microplate ELISA, respectively, as described elsewhere (Åhren *et al*., 1993, Vaccine 11, 929). The anti-LT antibody titres were divided by the total IgA concentration in the sample to yield the anti-LT IgA index which adjusts for variations in the total IgA content in the fecal samples collected from different children.

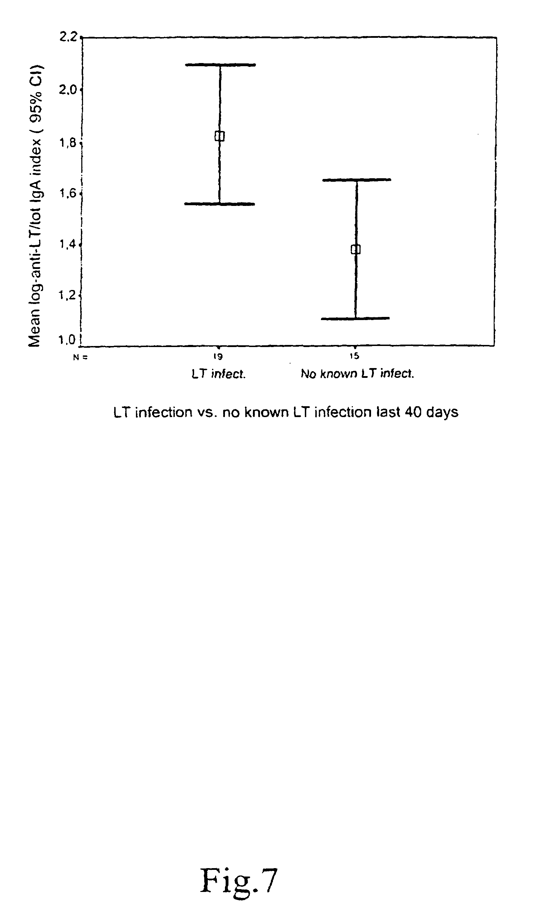

Figure 7 shows the mean and 95% confidence intervals of the log transformed anti-LT IgA indices in children known and not known to be infected with LT-ETEC during the last 40 days, as identified by examination of weekly collected stool samples with DNA-DNA hybridization for LT-ETEC. The fold-difference in the geometric mean anti-LT IgA indices between the two groups was 2.8; the t-test for this difference yielded a p value of 0.02 while the non-parametric Mann-Whitney U-test yielded a p value of 0.03, showing that the children known to be infected with LT-ETEC dunng the last 40 days had significantly higher anti-LT IgA indices than those not known to have been infected. It should be noted that as the stool examinations were performed only at weekly intervals, it is likely that some of the 15 children not known to have been infected with LT-ETEC were indeed infected with this very commonly occurring pathogen. Moreover, as cholera was prevalent during the study period, many of the study children were likely to have received breast milk containing not only anti-LT antibodies, but also anti-cholera toxin antibodies, which are detected in the employed ELISA. Accordingly, the observed difference in geometric mean titres between the two groups of children is likely to be an underestimate. Such differences would not have been possible to detect without an extensive protection from coproantibody breakdown.

This indicates that identifying fecal antibody responses is a useful tool in studying immune responses to natural infections as well as to enteric vaccines. To be used in a diagnostic test system, especially under such endemic conditions, pre- and post-infection antibody levels should be measured. Such pre-post comparison was not undertaken in the present study. Taking these considerations into account, the ability of our study to distinguish between the infected and apparently uninfected child groups was surprising and gives a conservative measure of the test's capacity to accurately identify zinc-gluconate-protected intestinal antibodies.

While this invention has been described with respect to particular embodiments thereof, it is apparent that numerous other forms and modifications of the invention will be obvious to those skilled in the art. The appended claims and this invention should be construed to cover all such obvious forms and modifications which are within the true spirit and scope of the present invention.

## Claims

1. A method of inhibiting the degradation of proteins in external secretions **characterized in that** the method comprising adding zinc gluconate to the external secretions.

2. The method of claim 1, **characterized in that** the concentration of zinc gluconate is in the range from 0.01 to 20 % by w/v, preferably from 5 to 15 % by w/v, and most preferable approximately 10 % by w/v.

3. The method of claim 1, **characterized in that** the proteins are immunoglobulins.

4. The method of claim 3, **characterized in that** the immunoglobulins are selected from the group consisting of IgG, IgA, IgM, IgD and IgE.

5. The method of claim 4, **characterized in that** the immunoglobulins are antibodies selected from the group consisting of antibodies to enteric pathogens such as *V. cholerae, S.typhi, Shigella* species, *Salmonella, E. histolytica, G. lamblia*, diarrhoegenic *E. coli*, as well as antibodies to Helicobacter pylori, rotavirus, HIV-1, hepatitis A, hepatitis B, hepatitis E, hepatitis F, food antigens including gluten and allergens.

6. The method of claim 1, **characterized in that** the external secretions are selected from the group consisting of feces, urine, saliva, nasal secretions and vaginal secretions.

7. The method of one of the preceding claims **characterized in that** the solution further comprising one or more preservatives such as anti-bacterial agents, anti-fungal agents, bacteriostatic agents, fungistatic agents and enzyme inhibitors.

8. The use of zinc gluconate in an immunological method for analyzing immunoglobulins in a fecal sample, wherein immediately upon collection the zinc gluconate is added to and mixed with the fecal sample in order to inhibit the degradation of the immunoglobulins and thus enabling the recovery of high concentrations of immunoglobulins, and whereafter the immunogloblulins are analyzed by enzyme-linked immunoabsorbent assay (ELISA) or any other assay for the detection of immunoglobulins such as radioimmuno assays, Western Blot assay etc.

9. A device (10,10',10") for collecting and storing substances from a feces sample for subsequent testing **characterized in that** the device (10,10',10") comprises a container (12,12') comprising zinc gluconate, said container (12,12') being closed by a cap or lid (16,16',16").

10. The device (10) of claim 9, **characterized in that** the device (10) further comprises a spoon (20) attached to a lid (22) adapted to fit into and seal the container (12).

11. The device (10) of claim 9 or 10 **characterized in that** the container (12) is provided with a membrane (18).

12. The device (10') of claim 9 **characterized in that** the device (10') further comprises a foldable or collapsible spoon (20') attached to the lid (16') adapted to fit into and seal the container (12').

13. The device (10') of claim 12 **characterized in that** the container (12') is provided with a membrane (18').

14. The device (10") of claim 9, **characterized in that** the device has the form of a syringe (10") provided with a plurality of compartments (22") (chambers) wherein zinc gluconate is contained in one of the compartments, and wherein the piston (24") while pulled backwards to suck the fecal sample into the syringe will break the separation membrane(s) (26") between the independent compartments (22").

15. Device (10") of claim 14, **characterized in that** one of the compartments (22") comprises zinc gluconate in dry form and another compartment (22") comprises a suitable buffer solution, such as PBS.

16. Device (10") of claim 13 or 14, **characterized in that** the syringe (10") further comprises an empty compartment (22").

## Patentansprüche

1. Verfahren zur Inhibierung des des Abbaus von Proteinen in externen Ausscheidungen, **dadurch gekennzeichnet, daß** das Verfahren das Zusetzen von Zinkgluconat zu den externen Ausscheidungen umfaßt.

2. Verfahren nach Anspruch 1, **dadurch gekennzeichnet, daß** die Zinkgluconatkonzentration im Bereich von 0,01 bis 20 % (Gew./Vol.), bevorzugt im Bereich von 5 bis 15 % (Gew./Vol.) liegt und am meisten bevorzugt etwa 10 % (Gew./Vol.) beträgt.

3. Verfahren nach Anspruch 1, **dadurch gekennzeichnet, daß** die Proteine Immunoglobuline sind.

4. Verfahren nach Anspruch 3, **dadurch gekennzeichnet, daß** die Immunoglobuline ausgewählt sind aus der Gruppe bestehend aus IgG, IgA, IgM, IgD und IgE.

5. Verfahren nach Anspruch 4, **dadurch gekennzeichnet, daß** die Immunoglobuline Antikörper, ausgewählt aus der Gruppe bestehend aus Antikörpern gegen enterische Pathogene wie z.B. *V. cholerae, S.typhi, Shigella* species, *Salmonella, E. histolytica, G. lamblia,* durchfallerregende *E. coli,* sowie Antikörper gegen Helicobacter pylori, Rotavirus, HIV-1, Hepatitis A, Hepatitis B, Hepatitis E, Hepatitis F, Lebensmittelantigene, einschließlich Gluten, und Allergene sind.

6. Verfahren nach Anspruch 1, **dadurch gekennzeichnet, daß** die externen Ausscheidungen ausgewählt sind aus der Gruppe bestehend aus Fäzes, Urin, Speichel, Nasensekrete und Vaginalsekrete.

7. Verfahren nach einem der vorhergehenden Ansprüche, **dadurch gekennzeichnet, daß** die Lösung außerdem ein oder mehrere Konservierungsmittel, wie z.B. antibakterielle Mittel, Anti-Pilzmittel, bakteriostatische Mittel, fungistatische Mittel und Enzyminhibitoren, umfaßt.

8. Anwendung von Zinkgluconat in einem immunologischen Verfahren zur Analyse von Immunoglobulinen in einer Fäzesprobe, bei welchem Verfahren das Zinkgluconat sofort nach Einsammlung der Fäzesprobe dieser zugesetzt und mit ihr vermischt wird um den Abbau der Immunoglobuline zu inhibieren und somit die Herstellung von hohen Konzentrationen von Immunoglobulinen zu ermöglichen, wonach die Immunoglobuline mittels eines Enzym-gebundenen immunosorbent-Tests (ELISA) oder eines jeden anderen Tests zur Detektierung von Immunoglobulinen, wie z.B. Radioimmun-Tests, Western Blot usw. analysiert werden.

9. Vorrichtung (10,10',10") für die Einsammlung und Aufbewahrung von Stoffen einer Fäzesprobe im Hinblick auf ein späteres Testen, **dadurch gekennzeichnet, daß** die Vorrichtung (10,10',10") einen das Zinkgluconat umfassenden Behälter (12,12') umfaßt, wobei der Behälter (12,12') mit einer Haube oder einem Deckel (16,16',16") geschlossen ist.

10. Vorrichtung (10) nach Anspruch 9, **dadurch gekennzeichnet, daß** die Vorrichtung (10) außerdem einen Löffel (20) umfaßt, welcher Löffel an einem Deckel (22) befestigt ist, welcher derart ausgefertigt ist, daß er in den Behälter (12) paßt und diesen versiegelt.

11. Vorrichtung (10) nach Anspruch 9 oder 10, **dadurch gekennzeichnet, daß** der Behälter (12) mit einer Membrane (18) versehen ist.

12. Vorrichtung (10') nach Anspruch 9, **dadurch gekennzeichnet, daß** die Vorrichtung (10') außerdem einen faltbaren oder zusammenklappbaren Löffel (20') umfaßt, welcher Löffel an dem Deckel (16') befestigt ist, welcher derart ausgefertigt ist, daß er in den Behälter (12') paßt und diesen versiegelt.

13. Vorrichtung (10') nach Anspruch 12, **dadurch gekennzeichnet, daß** der Behälter (12') mit einer Membrane (18') versehen ist.

14. Vorrichtung (10") nach Anspruch 9, **dadurch gekennzeichnet, daß** die Vorrichtung die Form einer Spritze (10") mit einer Vielzahl von Abteilungen (22") (Kammern) hat, wobei eine der Abteilungen Zinkgluconat enthält, und wobei der Kolben (24"), wenn dieser zum Einsaugen der Fäzesprobe in die Spritze zurückgezogen wird, die Trennmembrane(n) (26") zwischen den unabhängigen Abteilungen (22") durchbricht.

15. Vorrichtung (10") nach Anspruch 14, **dadurch gekennzeichnet, daß** eine der Abteilungen (22") Zinkgluconat in trockener Form umfaßt, und eine andere Abteilung (22") eine passende Pufferlösung, wie z.B. eine phosphatgepufferte Salzlösung (PBS), umfaßt.

16. Vorrichtung (10") nach Anspruch 13 oder 14, **dadurch gekennzeichnet, daß** die Spritze (10") außerdem eine leere Abteilung (22") umfaßt.

## Revendications

1. Méthode d'inhibition de la décomposition de protéines dans les excrétions, **charactérisée en ce que** la méthode comprend l'addition de gluconate de zinc aux excrétions.

2. Méthode selon la revendication 1, **charactérisée en ce que** la concentration du gluconate de zinc est de 0,01 à 20 % (poids/volume), de préférence de 5 à 15 % (poids/volume) et tout préférentiellement de 10 % (poids/volume) environ.

3. Méthode selon la revendication 1, **charactérisée en ce que** les protéines sont des immunoglobulines.

4. Méthode selon la revendication 3, **charactérisée en ce que** les immunoglobulines sont choisies dans le groupe constitué par IgG, IgA, IgM, IgD et IgE.

5. Méthode selon la revendication 4, **charactérisée en ce que** les immunoglobulines sont des anticorps choisis dans le groupe constitué d'anticorps contre les pathogènes entériques tels que *V. cholerae, S.typhi, Shigella* species, *Salmonella, E. histolytica, G. lamblia, E. coli* agent diarrhéique, ainsi que des anticorps contre l'Helicobacter pylori, le rotavirus, le VIH-1, l'hépatite A, l'hépatite B, l'hépatite E, l'hépatite F, des antigène d'aliments, y-compris le gluten, et des allergènes.

6. Méthode selon la revendication 1, **charactérisée en ce que** les excrétions sont choisies dans le groupe constitué de fèces, urine, salive, secrétions nasales et secrétions vaginales.

7. Méthode selon l'une des revendications précédentes, **charactérisée en ce que** la solution comprend en outre un ou plusieurs agents conservateurs tels que les agents antibactériens, les agents antifongiques, les agents bactériostatiques, les agents fongistatiques et les inhibiteurs d'enzyme.

8. Utilisation de gluconate de zinc dans une méthode immunologique pour l'analyse d'immunoglobulines dans un échantillon fécal, dans quelle méthode le gluconate de zinc est additionné à et mélangé avec l'échantillon fécal immédiatement après sa collection en vue d'inhiber la décomposition des immunoglobulines et ainsi permettant la récupération de concentrations élevées d'immunoglobulines, après quoi les immunoglobulines sont analysées par essai d'immuno-absorption enzymatique (ELISA) ou par tout autre essai de détection d'immunoglobulines, tels les radio-immuno-essais, le Western Blot etc.

9. Dispositif (10,10',10") pour la collection et le stockage de substances d'un échantillon fécal en vue d'un essai subséquent, **charactérisé en ce que** le dispositif (10,10',10") comprend un conteneur (12,12') comprenant le gluconate de zinc, ledit conteneur (12,12') étant fermé par une chape ou par un couvercle (16,16',16").

10. Dispositif (10) selon la revendication 9, **charactérisé en ce que** le dispositif (10) comprend en outre une cuillère (20) étant attachée à un couvercle (22), qui est adapté pour s'insérer dans le conteneur (12) et le sceller.

11. Dispositif (10) selon la revendication 9 ou 10, **charactérisé en ce que** le conteneur (12) est pourvu d'une membrane (18).

12. Dispositif (10') selon la revendication 9, **charactérisé en ce que** le dispositif (10') comprend en outre une cuillère pliable ou pliante (20') étant attachée au couvercle (16') qui est adapté pour s'insérer dans le conteneur (12') en le scellant.

13. Dispositif (10') selon la revendication 12, **charactérisé en ce que** le conteneur (12') est pourvu d'une membrane (18').

14. Dispositif (10") selon la revendication 9, **charactérisé en ce que** le dispositif a la forme d'une seringue (10") pourvue d'une pluralité de compartiments (22") (chambres), le gluconate de zinc étant contenu dans un des compartiments, et le piston (24") allant rompre la/les membrane(s) (26") entre les compartiments indépendants (22") lorsqu'il est retiré en vue d'aspirer l'échantillon fécal dans la seringue.

15. Dispositif (10") selon la revendication 14, **charactérisé en ce que** l'un des compartiments (22") comprend du gluconate de zinc sous forme sèche et qu'un autre compartiment (22") comprend une solution tampon convenable telle qu'une solution saline tamponnée au phosphate (PBS).

16. Dispositif (10") selon la revendication 13 ou 14, **charactérisé en ce que** la seringue (10") comprend en outre un compartiment vide (22").
